# EUROPEAN PATENT APPLICATION

(11) **EP 1 491 081 A1**
(43) Date of publication of application: **29.12.2004**
(21) Application number: 04253708.4
(22) Date of filing: 21.06.2004
(51) Int. Cl.: A01C 1/06, A01H 4/00

(54) **Method of creating an end seal for a manufactured seed**

(30) Priority: 24.06.2003 US 560711 P
(71) Applicant: Weyerhaeuser Company, Federal Way, WA 98063-9777 (US)
(72) Inventor: Hirahara, Edwin, Federal Way Washington 98023 (US)
(74) Representative: Bayliss, Geoffrey Cyril

(57) **Abstract**

A method of forming an end seal (50) for a manufactured seed (22) is provided. The method includes providing a manufactured seed having an open-end (56) and immersing at least the open end of the manufactured seed in a sealing material (54) for a predetermined period of time. The method also includes permitting the sealing material to harden over the opened end of the manufactured seed to form a plug that seals the opened end.

## Description

The present invention relates generally to manufactured seeds and, more particularly, to a method of creating an end seal for a manufactured seed.

Asexual propagation for plants has been shown for some species to yield large numbers of genetically identical embryos, each having the capacity to develop into a normal plant. Such embryos must usually be further cultured under laboratory conditions until they reach an autotrophic "seedling" state characterized by an ability to produce their own food via photosynthesis, resist desiccation, produce roots able to penetrate soil and fend off soil microorganisms. Some researchers have experimented with the production of the artificial seeds, known as manufactured seeds, in which individual plant somatic or zygotic embryos are encapsulated in a seed coat. Examples of such manufactured seeds are disclosed in U.S. Patent No. 5,701,699, issued to Carlson et al., the disclosure of which is hereby expressly incorporated by reference.

Typical manufactured seeds include a seed coat, synthetic gameophyte and a plant embryo. The seed coat is suitably a cylindrical capsule having a closed end formed by a primary end seal and an open end. The synthetic gameophyte is placed within the seed coat and substantially fills the interior of the seed coat. A longitudinally extending hard porous insert, commonly known as a cotyledon restraint, may be centrally located within the synthetic gameophyte. The cotyledon restraint includes a centrally located cavity extending partially through the length of the cotyledon restraint and is sized to receive the plant embryo.

The well-known plant embryo includes a radicle end and a cotyledon end. The plant embryo is deposited within the cavity of the cotyledon restraint cotyledon end first. The plant embryo is then sealed within the seed coat by at least one secondary end seal. There is a weakened spot in the secondary end seal to allow the radicle end of the embryo to penetrate the secondary end seal.

In the past, crimping the sidewalls of the seed coat formed the end seal. Forming the end seal by crimping the sidewalls resulted in a seal that was not uniform in shape and usually allowed for undesirable variations in the internal depth of the seed coat. Further, such crimping methods did not create a reliable seal and typically broke as the seed coat was subjected to mechanical manipulation. The creation of the crimp was a process that requires a fairly significant amount of time. Moreover, such a process requires an empty seed coat because a die must be inserted into the seed coat upon which the crimp was formed. In addition, the formation of the crimp requires a secondary operation that flattens the folds of the crimp against the die to complete the crimping process.

In accordance with one embodiment of the present invention, a method of forming an end seal for a manufactured seed is provided. The method includes providing a manufactured seed having an open end, and immersing at least the open end of the manufactured seed in a sealing material for a predetermined period of time. The method also includes permitting the sealing material to harden over the open end of the manufactured seed to form a plug that seals the open end.

In another embodiment of the present invention, the sealing material is a wax. Further, the predetermined period of time is substantially between a range of 0.1 and 10 seconds. In certain embodiments of the present invention, the manufactured seed is immersed in the sealing material for a total of one second.

The method of forming an end seal in accordance with the embodiments of the present invention has several advantages over currently available methods. As a non-limiting example, the method of the present invention provides a stronger and more reliable seal even under extreme mechanical manipulation. Thus, a method of forming an end seal in accordance with the present application results in an end seal that is more reliable, faster, and stronger than end seals formed by existing methods and can be applied to empty seed coats or seed coats filled with synthetic gametophyte and with a cotyledon restraint.

The foregoing aspects and many of the attendant advantages of this invention is best understood by reference to the following detailed description, when taken in conjunction with the accompanying drawings, wherein:
FIGURE 1 is a cross-sectional side view of a manufactured seed with an end seal formed in accordance with one embodiment of the present invention; and
FIGURE 2 is a cross-sectional side view of a seed coat for a manufactured seed and a reservoir of wax used to form an end seal.

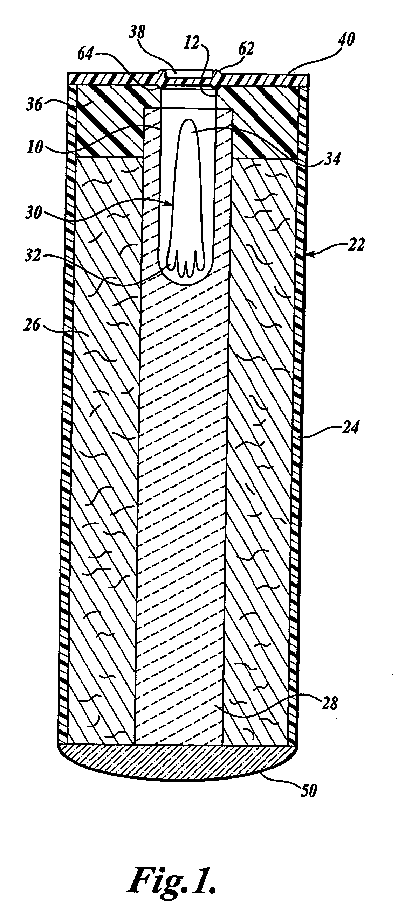

FIGURE 1 illustrates an end seal 50 constructed in accordance with the present invention for a manufactured seed 22. As is disclosed in U.S. Patent No. 5,701,699, issued to Carlson et al., the disclosure which is hereby incorporated by reference, well known manufactured seeds 22 include a seed coat 24, synthetic gametophyte 26, a cotyledon restraint 28, a plant embryo 30, a primary end seal 36, and a secondary end seal 40. The cotyledon restraint 28 is suitably manufactured from a hard porous material and includes a longitudinally extending cavity 10. The cavity 10 extends through the primary end seal 36 and partially through one end of the cotyledon restraint 28. The open end of the cavity 10 is known as a cotyledon restraint opening 12. The cavity 10 is sized to receive the plant embryo 30 therein. The plant embryo 30 includes a cotyledon end 32 and a root end 34.

The secondary end seal 40 seals the cotyledon restraint opening 12. The end seal 40 is suitably formed from a sheet of polymeric film and includes a prestressed area 38. The prestressed area 38 is centrally located above the cotyledon restraint opening 12, as is disclosed in greater detail below. Such an end cap 40 is disclosed in U.S. Patent No. 6,470,623, issued to Hirahara, the disclosure of which is hereby expressly incorporated by reference.

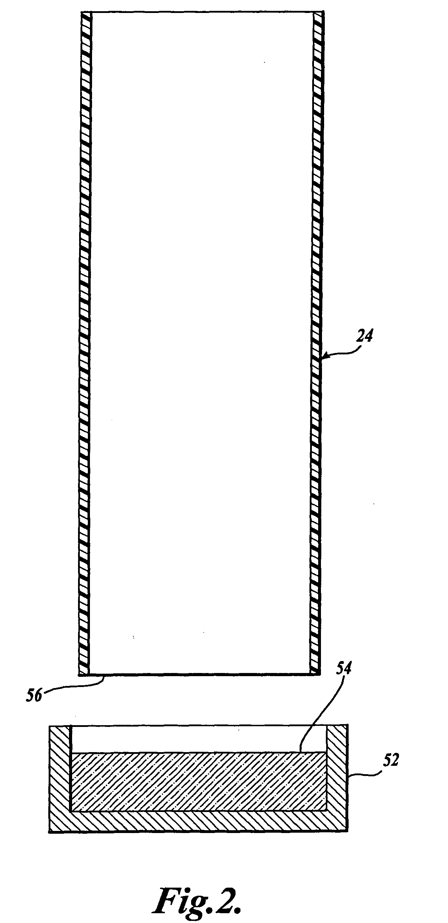

Manufacture and attachment of the end seal 50 may be best understood by referring to FIGURE 2. Preferably, the end seal 50 is formed after the seed coat 24 has been filled with at least the gametophyte 26 and cotyledon restraint 28. In other embodiments, the end seal 50 is suitably formed before the seed coat 24 is filled with any material, such as the gametophyte 26 and cotyledon restraint 28. In both embodiments, the seed coat 24 is positioned above a container 52 filled with a thermoplastic sealing material 54, such as wax or wax mixture. For ease of illustration, FIGURE 2 depicts an empty seed coat 24. It should be apparent that a filled seed coat 24 is also within the scope of the present invention.

In certain embodiments, the container 52 is heated to raise the temperature of the sealing material 54. In such embodiments, the sealing material 54 in its solid state is placed within the container 52. Heat is applied to the container 52 until the sealing material 54 changes state to a fluid. In other embodiments, the sealing material 54 may liquefied outside of the container 52 and then poured into the container 54.

Although wax is the preferred sealing material, additional types of sealing materials are also within the scope of the present invention. As a non-limiting example, any thermoplastic material capable of changing states in response to a change in temperature, such as plastic, is also within the scope of the present invention.

An open end 56 of the seed coat 24 is immersed within the sealing material 54 for a predetermined period of time. The predetermined period of time, sealing material 54 used, and temperature of the sealing material 54 when it is disposed within the container 52, all affect the shape and thickness of the resulting end seal 50. Each of the foregoing parameters may be varied to control the shape and thickness of the end seal 50. As a non-limiting example, if the seed coat 24 is soaked in the sealing material 54 for a period of time exceeding the time it takes for the sealing material 54 to adhere to the open end of the seed coat 24, the sealing material 54 flows off the end of the end of the seed coat 24 to create a nipple-like protrusion.

The length of time the seed coat 24 is immersed within the container 52 is determined by observation. Specifically, after the seed coat 24 is removed from the container 52, the resulting end seal 50 is observed to ensure that a proper seal between the end seal 50 and the seed coat 24 has been formed. In certain embodiments of the present invention, the predetermined period of time may range between 0.1 seconds and 50 seconds, or longer. In another embodiment, the range is between 0.1 and 8 seconds. In still yet another embodiment, the seed coat 24 is immersed within the sealing material 54 in a range between 0.5 to 5 seconds. In yet another non-limiting example, the seed coat 24 is immersed in the sealing material 54 for one second. It should be apparent to one of ordinary skill that the soak time may be shorter or longer than those set forth above, and therefore, such times are also within the scope of the present invention.

It has been discovered that depending on how long the seed coat 24 is immersed within the sealing material 54, the depth to which the seed coat 24 is immersed within the sealing material 54, and the density and temperature of the sealing material 54, all combine to determine whether a seal is formed and also determines the shape of the end seal 50. Thus, adjusting various parameters, such as the time that the seed coat 24 is immersed within the sealing material 54, results in end seals 50 of varying thickness and shapes.

After the seed coat 24 has soaked within the sealing material 54 for the predetermined period of time, the seed coat 24 is removed from the container 52. The sealing material 54 then drips down and off the seed coat 24 and the remaining sealing material 54 hardens to seal the open end 56 of the seed coat 24. The sealing material 54 forms a plug that is attached to both the sidewalls and the ends of the seed coats 24.

While the preferred embodiment of the invention has been illustrated and described, it will be appreciated that various changes can be made therein without departing from the spirit and scope of the invention. As a non-limiting example, it should be apparent to one of ordinary skill in the art that forming an end seal 50 in accordance with embodiments of the present invention may be incorporated with an automated system. In other applications, the seed coat 24 may be manually dipped and soaked in the sealing material 54. Thus, both automatic and manual methods of dipping the seed coats 24 within the container 52 are within the scope of the present invention.

## Claims

1. A method of forming an end seal for a manufactured seed, comprising:
(a) providing a manufactured seed coat having an open end;
(b) immersing at least the open end of the manufactured seed coat in a sealing material for a predetermined period of time; and
(c) permitting the sealing material to harden over the open end of the manufactured seed coat to form a plug that seals the open end.

2. A method of forming an end seal for a manufactured seed as claimed in claim 1, wherein the sealing material is a wax.

3. A method of forming an end seal for a manufactured seed as claimed in claim 1 or claim 2, wherein the plug is curved in cross-section.

4. A method of forming an end seal for a manufactured seed as claimed in any of claims 1 to 3, wherein the predetermined period of time is substantially within a range of 0.1 to 15 seconds.

5. A method of forming an end seal for a manufactured seed, comprising:
(a) providing a container having a wax;
(b) inserting a manufactured seed having an open end into the container having a wax; and
(c) removing the manufactured seed from the container, the wax forming a plug at the open end of the manufactured seed to seal the open end of the manufactured seed.

6. A method of forming an end seal for a manufactured seed as claimed in claim 5, wherein removing the manufactured seed occurs within a predetermined period of time.

7. A method of forming an end seal for a manufactured seed as claimed in claim 6, wherein the predetermined period of time is substantially within a range of 0.1 to 15 seconds.

8. A method of forming an end seal for a manufactured seed as claimed in claim 6 or claim 7, wherein the predetermined period of time is substantially within a range of 0.5 to 5 seconds.

9. A method of forming an end seal for a manufactured seed, comprising:
(a) filling a container with a liquid wax;
(b) inserting an open end of a manufactured seed coat into the container;
(c) soaking the open end of the manufactured seed coat, such that at least a portion of the liquid wax attaches to the open end;
(d) removing the manufactured seed coat from the container; and
(e) permitting the liquid wax attached to the open end to harden and seal the open end of the manufactured seed coat.

10. A method of forming an end seal for a manufactured seed as claimed in claim 9, wherein removing the open end of the manufactured seed coat from the containers occurs within a predetermined period of time.

11. A method of forming an end seal for a manufactured seed as claimed in claim 10, wherein the predetermined period of time is substantially within a range of 0.1 to 15 seconds.

12. A method of forming an end seal for a manufactured seed as claimed in claim 10 or claim 11, wherein the predetermined period of time is substantially within a range of 0.5 and 5 seconds.

13. A method of forming an end seal for a manufactured seed as claimed in any of claims 10 to 12, wherein the predetermined period of time is substantially one second.

14. An end seal for a manufactured seed substantially as described with reference to and as illustrated in the accompanying drawings.
